# FASCICULE DE BREVET EUROPEEN

(11) **EP 0 793 484 B1**
(45) Date de publication et mention de la délivrance du brevet: **19.02.2003**
(21) Numéro de dépôt: 95940309.8
(22) Date de dépôt: 14.11.1995
(51) Int. Cl.: A61K 9/127, A61K 47/28, A61K 39/39

(54) **ADJUVANT POUR COMPOSITION VACCINALE**
ADJUVANS FÜR IMPFSTOFF
ADJUVANT FOR A VACCINE COMPOSITION

(30) Priorité: 14.11.1994 FR 9413606
(43) Date de publication de la demande: 10.09.1997
(73) Titulaire: Aventis Pasteur, 69367 Lyon Cedex 07 (FR)
(72) Inventeur: HAENSLER, Jean, 69290 St Genis-les-Ollières (FR); TRANNOY, Emmanuelle, 69005 Lyon (FR); RONCO, Jorge, 69350 LA MULATIERE (FR)
(74) Mandataire: Kerneis, Danièle
(86) Numéro de dépôt international: FR9501495
(87) Numéro de publication internationale: WO96014831

(56) Documents cités:
- EP-A- 0 356 339
- WO-A-93/05162
- WO-A-93/14744
- US-A- 4 261 975
- US-A- 4 971 803
- JOURNAL OF LIPOSOME RESEARCH, vol. 4, no. 3, Novembre 1994 MONZICELLO (US), pages 1075-1090, XP 000476607 K.M. HUI ET AL. 'induction of alloreactive cytotoxic T lymphocytes by intra-splenic immunization with allogeneic class I Major Histocompatibility complex DNA and Dc-chol cationic liposomes'
- Gupta et al "Adjuvants - a balance between toxicity and adjuvanticity", Vaccine, Vol. 11, 1993, p. 293
- Li et al. "DC-Chol lipid system in gene transfer", Journal of Controlled Release, Vol. 39, 1996, p. 373-381

## Description

La présente invention est relative au domaine des compositions vaccinales. Plus particulièrement, l'invention concerne de nouveaux adjuvants utilisés pour augmenter l'immunogénicité de compositions vaccinales.

Il existe une grande quantité d'antigènes qui, injectés chez l'animal, vont provoquer une fabrication d'anticorps qui leur sont spécifiques. Un des principes de la vaccination est de stimuler la fabrication d'anticorps par l'organisme d'un homme ou d'un animal, en lui administrant des antigènes choisis. Les anticorps ainsi fabriqués permettront, ensuite, à l'organisme de se défendre contre une infection ultérieure. Cependant, certains antigènes n'entraînent pas de stimulation suffisante du système immunitaire lorsqu'ils sont administrés seuls. Il est donc nécessaire de leur adjoindre un adjuvant qui va permettre d'augmenter la réponse immunitaire de l'organisme afin d'obtenir une quantité d'anticorps suffisante pour être protectrice.

Parmi l'art antérieur relatif aux adjuvants, on peut citer la demande de brevet EP356339 qui décrit des liposomes susceptibles d'être des adjuvants pour l'antigène de la grippe; aucun produit utilisant ces liposomes n'est cependant aujourd'hui commercialisé.

Parmi les adjuvants connus, on peut citer l'hydroxyde d'aluminium et le phosphate d'aluminium qui sont utilisés habituellement dans les vaccins humains. Cependant, ces composés ne possèdent pas de propriété adjuvante vis-à-vis de tous les antigènes. Ils ne permettent notamment pas d'augmenter l'immunogénicité du vaccin contre la grippe.

Il est donc nécessaire de pouvoir disposer d'adjuvants qui permettent d'augmenter l'immunogénicité des antigènes administrés dans une composition vaccinale, sans aucun risque de toxicité.

En outre, il est intéressant de disposer d'adjuvants capables d'induire une réponse immunitaire se traduisant par une production d'anticorps sécrétoires, tels que des IgA.

Pour atteindre ce but, l'invention propose l'utilisation d'un composé amphipathique comprenant un groupement lipophile dérivé d'un stérol lié à un groupement cationique pour la fabrication d'une composition vaccinale.

L'invention a aussi pour objet l'utilisation d'un tel composé amphipathique pour la fabrication d'un adjuvant pour une administration vaccinale.

L'invention a également pour objet une composition vaccinale comprenant au moins un antigène, caractérisée en ce qu'elle comprend en outre au moins un composé amphipathique possédant un groupement lipophile dérivé d'un stérol lié à un groupement cationique.

Au sens de la présente invention, le terme amphipathique désigne un composé qui possède à la fois une portion hydrophobe et une portion hydrophile.

Parmi les dérivés de stérol pouvant conduire aux composés selon l'invention, on peut citer le cholestérol, le phytostérol, l'ergostérol. Les dérivés de cholestérol conviennent particulièrement bien.

Le groupement cationique des composés amphipathiques selon l'invention peut être constitué par un ammonium quaternaire ou une amine protonable.

Le groupement lipophile est relié au groupement cationique grâce à une liaison ester, éther, amide ou carbamoyle parmi lesquelles les liaisons ester, amide et carbamoyle présentent l'avantage d'être hydrolysables dans la cellule.

La liaison entre les 2 groupements est réalisée de préférence par l'intermédiaire d'un bras espaceur constitué par une chaîne alkylique ramifiée ou non comprenant de 1 à 20 atomes de Carbone.

Parmi les composés convenant aux fins de l'invention, on peut citer ceux mentionnés dans le brevet US 5 283 185 et notamment :
- l'iodure de cholestéryl-3β-carboxamidoéthylènetriméthylammonium,
- la cholestéryl-3β-carboxyamidoéthylènamine,
- l'iodure de cholestéryl-3β-oxysuccinamidoéthylènetriméthylammonium,
- le 3β-[N-(N',N'-diméthylaminoéthane)-carbamoyl]-cholestérol,
- le 3β-[N-(polyéthylènimine)-carbamoyl]-cholestérol.
parmi lesquels le 3β-[N-(N',N'-diméthylaminoéthane)-carbamoyl]-cholestérol est particulièrement avantageux.

Les composés amphipathiques selon l'invention peuvent être obtenus par condensation entre un dérivé de stérol et un composé à groupement cationique. selon une des méthodes décrites dans "Advanced Organic Chemistry" Part B : Reactions and Synthesis (F.A. Carey and R.J. Sundberg - Plenum Publishing Corp.). Plus particulièrement, on peut réaliser certains des composés selon l'invention selon les méthodes décrites dans le brevet US 5 283 185.

Les composés amphipathiques obtenus en solution alcoolique peuvent, ensuite, être dispersés dans de l'eau ou dans un tampon aqueux et conduire à une suspension de micelles ou de liposomes. Avantageusement, les composés amphipathiques de l'invention sont associés à un lipide neutre tel qu'un phospholipide, par exemple la dioleoyl phosphatidyléthanolamine (DOPE) ou la dioleoyl phosphatidylcholine (DOPC). Cette association conduit les composés amphipathiques selon l'invention à s'organiser sous forme de liposomes plutôt que de micelles lors de la phase de dispersion dans un environnement aqueux. La proportion molaire de lipide neutre associé aux composés amphipathiques est, de préférence, supérieure à 20 %.

Les produits obtenus selon l'invention n'ont conduit à aucune réaction de toxicité aigüe lors de leur inoculation à des souris.

L'antigène utilisé pour induire une réponse immunitaire protectrice est constitué par n'importe quel antigène utilisé habituellement dans une composition vaccinale, que ce soit seul ou en combinaison avec un autre antigène.

De façon particulière, les composés amphipathiques selon l'invention se révèlent de bons immuno-adjuvants lorsqu'ils sont associés au vaccin contre le virus de la grippe qui comprend notamment : la protéine HA qui est une hémagglutinine située à la surface de l'enveloppe du virus Influenza, la protéine NP qui est une nucléoprotéine de capside liée à l'ARN viral et une protéine M ou "matrice" protéique de l'enveloppe.

L'association entre l'antigène dont on veut accroître l'immunogénicité et la suspension micellaire ou liposomale de composés amphipathiques est réalisée spontanément par interaction hydrophobe et électrostatique lors du mélange des constituants.

Les compositions vaccinales obtenues présentent une bonne stabilité. Cependant, la suspension liposomale semble préférable à la suspension micellaire.

En outre, la suspension liposomale est stérilisable par filtration et lyophilisable.

Il est évident qu'il est possible d'ajouter aux compositions vaccinales obtenues des éléments utilisés classiquement dans les vaccins, tels que de l'eau, du sérum physiologique ou une substance tampon.

L'administration des compositions vaccinales obtenues selon l'invention peut être effectuée par toutes les voies habituellement utilisées pour l'administration de vaccins et, notamment, par voie sous-cutanée ou intranasale. Il est possible aussi de choisir pour la première immunisation et l'immunisation de rappel une voie différente.

Il est possible d'administrer, de façon séparée, la composition comprenant l'antigène et la composition contenant les composés amphipathiques selon l'invention ; cependant, l'administration d'une composition liposomale de composés amphipathiques selon l'invention associés à l'antigène permet non seulement d'augmenter la réponse immunitaire de type humorale, mais aussi d'induire des lymphocytes T cytotoxiques spécifiques.

L'invention sera mieux comprise à la lecture des exemples non limitatifs qui suivent, en référence aux figures.

La figure 1 illustre le schéma de réaction de la fabrication du DC chol. Les figures 2 à 6 représentent les résultats des tests d'induction des lymphocytes T cytotoxiques pour chaque groupe de souris mentionné à l'exemple 8.

La figure 7 représente les résultats mentionnés à l'exemple 11.

Les figures 8 et 9 représentent les résultats mentionnés à l'exemple 12.

Les figures 10 et 11 représentent les résultats mentionnés à l'exemple 13.

Les figures 12 et 13 représentent les résultats mentionnés à l'exemple 14.

Les figures 14 et 15 représentent les résultats mentionnés à l'exemple 15.

La figure 16 représente les résultats mentionnés à l'exemple 16.

### Exemple 1 : synthèse de 3β-[N-(N',N'-diméthylaminoéthane)-carbamoyl]-cholestérol (DC chol)

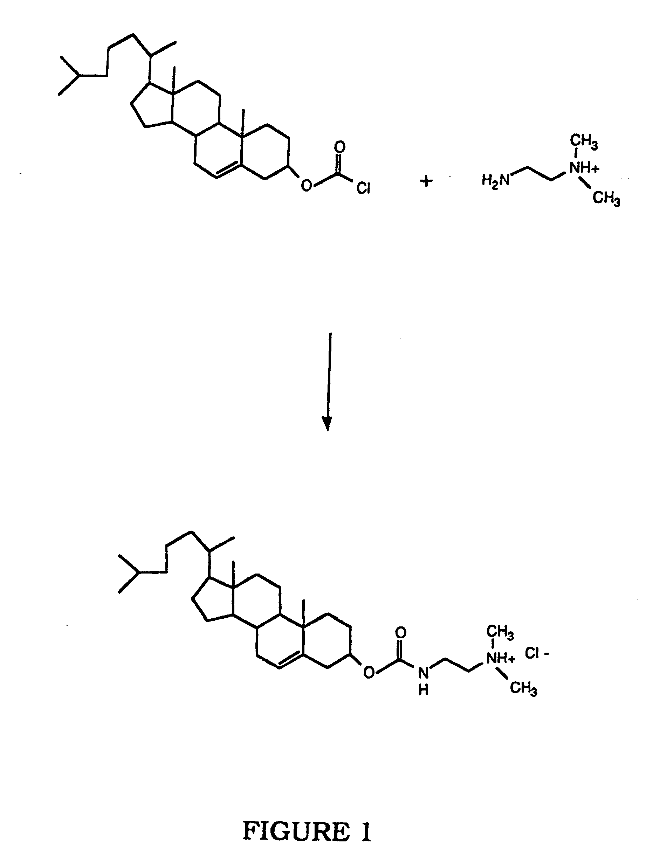

Le DC chol est synthétisé par réaction de cholestéryl chloroformate et de N,N-diméthyléthylènediamine selon le schéma de la figure 1, ainsi que cela est décrit dans l'article de X. Gao et L. Huang (BBRC 179 (1) : 280-285).

Une solution de cholestéryl chloroformate (2,25 g, 5 mmol dans 5 ml de chloroforme sec) est ajoutée goutte à goutte à une solution en excès de N,N-diméthyléthylènediamine (2 ml, 18.2 mmol dans 3 ml de chloroforme sec) à 0°C. Après extraction du solvant par évaporation, le résidu est purifié par 2 recristallisations successives dans de l'éthanol absolu à 4°C et séché sous vide. On obtient alors 0,545 g de DC chol sous forme de poudre blanche. La structure du composé a été vérifiée par RMN et spectrométrie de masse. Les résultats obtenus sont conformes aux données publiées dans l'article cité ci-dessus.

### Exemple 2 : Préparation d'une composition vaccinale contre le virus de la grippe, à partir d'une suspension micellaire de DC chol 2,3 mg

30 mg de DC chol obtenu selon l'exemple 1 sont dissous dans 100 µl d'éthanol. 75 µl de la solution ainsi obtenue sont injectés par l'intermédiaire d'une seringue Hamilton dans 3 ml d'eau maintenue sous agitation à 45°C. Après 5 minutes d'agitation supplémentaire à 45°C, la suspension micellaire obtenue est mélangée à 200 µl du vaccin monovalent contre le virus de la grippe (souche A Singapour) comprenant notamment comme antigènes : l'hémagglutinine HA, la nucléoprotéine NP et la protéine M.

Le mélange obtenu est fractionné en doses vaccinales de 0,3 ml. Chaque dose comprend 5µg de HA et 2,3 mg de DC chol.

### Exemple 3 : Préparation d'une composition vaccinale contre le virus de la grippe, à partir d'une suspension micellaire de DC chol 0,45 mg

On procède comme dans l'exemple 2 en partant de 6 mg de DC chol obtenu selon l'exemple 1.

### Exemple 4 : Préparation d'une suspension de liposomes constitués par du DC chol associé à de la dioleoyl phosphatidyléthanolamine (DOPE)

On mélange 18 mg de dioleoyl phosphatidyléthanolamine (DOPE) et 4,5 mg de DC chol obtenu selon l'exemple 1, que l'on met en solution dans 3 ml de chloroforme.

Le chloroforme est évaporé sous vide pour constituer un film lipidique qui est soumis à dessication sous vide, puis remis en suspension dans 3 ml d'eau.

Après hydratation pendant 24 heures à 4°C, la dispersion est soumise à sonication pendant 5 à 10 minutes dans un bain à ultra-sons (Laboratory Supplies - Hicksville - N.Y.) pour former des liposomes.

Cette suspension est stable pendant au moins 6 mois à 4°C.

### Exemple 5 : Préparation d'une suspension de liposomes consitués par du DC chol associé à de la dioleoyl phosphatidylcholine (DOPC)

On procède comme dans l'exemple 4 en remplaçant les 18 mg de DOPE par 18 mg de dioleoylphosphatidylcholine (DOPC).

On obtient une suspension liposomale stable pendant au moins 6 mois à 4°C.

### Exemple 6 : Préparation d'une composition vaccinale contre le virus de la grippe à partir d'une suspension liposomale DC chol/DOPE

3 ml d'une suspension liposomale obtenue selon l'exemple 4 sont mélangés à 0,2 ml du vaccin grippe monovalent de la souche A/ Singapour contenant l'équivalent de 50 µg de l'antigène constitué par l'hémagglutinine HA.

Le mélange obtenu est ensuite fractionné en 10 doses vaccinales de 0,3 ml, comportant chacune 5 µg de HA et 0,45 mg de DC chol.

### Exemple 7 : Préparation d'une composition vaccinale contre le virus de la grippe à partir d'une suspension liposomale DC chol/DOPC

3 ml d'une suspension liposomale obtenue selon l'exemple 5 sont mélangés à 0,2 ml du vaccin grippe monovalent de la souche A/ Singapour.

Le mélange obtenu est ensuite fractionné en 10 doses vaccinales de 0,3 ml comportant chacune 5 µg de HA et 0,45 mg de DC chol.

### Exemple 8 : Immunisation

On immunise 5 groupes de 4 souris Balb/c par 3 injections sous-cutanées effectuées à J0, J21 et J36 avec les compositions vaccinales suivantes :
Groupe A : 0,3 ml de vaccin grippe monovalent souche A/ Singapour dilué à 5 µg de HA dans 0,3 ml de PBS,
Groupe B : 0,3 ml de composition vaccinale obtenue selon l'exemple 2,
Groupe C : 0,3 ml de composition vaccinale obtenue selon l'exemple 3,
Groupe D : 0,3 ml de composition vaccinale obtenue selon l'exemple 6,
Groupe E : 0,3 ml de composition vaccinale obtenue selon l'exemple 7.

### Exemple 9 : Dosage en anticorps anti-HA

Afin d'effectuer les dosages en anticorps neutralisants, on prélève les sérums de souris à J21, J36 et J51 et on effectue le titrage en anticorps anti-HA grâce à la technique d'inhibition de l'hémagglutination induite par le virus de la grippe.

Le tableau 1 ci-dessous illustre les résultats obtenus pour chaque groupe de souris après une injection et le tableau 2 les résultats après 2 injections.

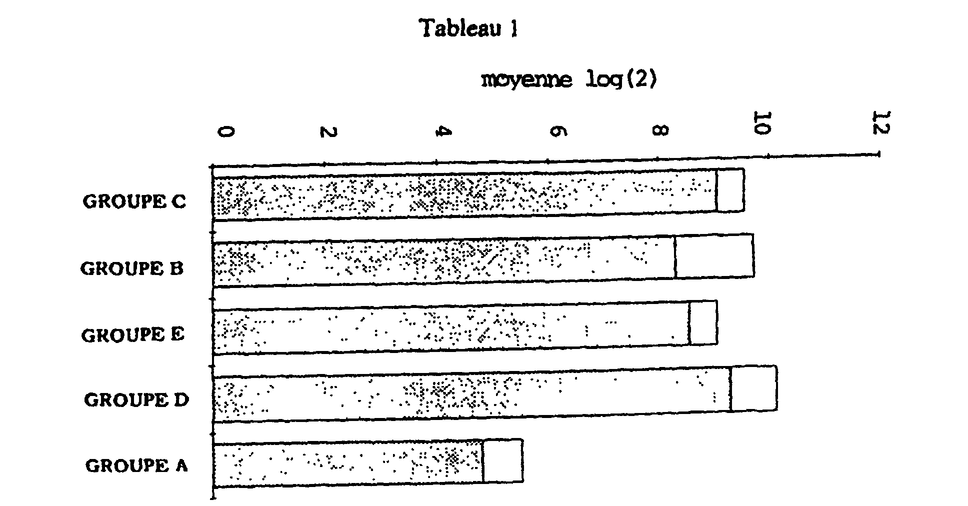

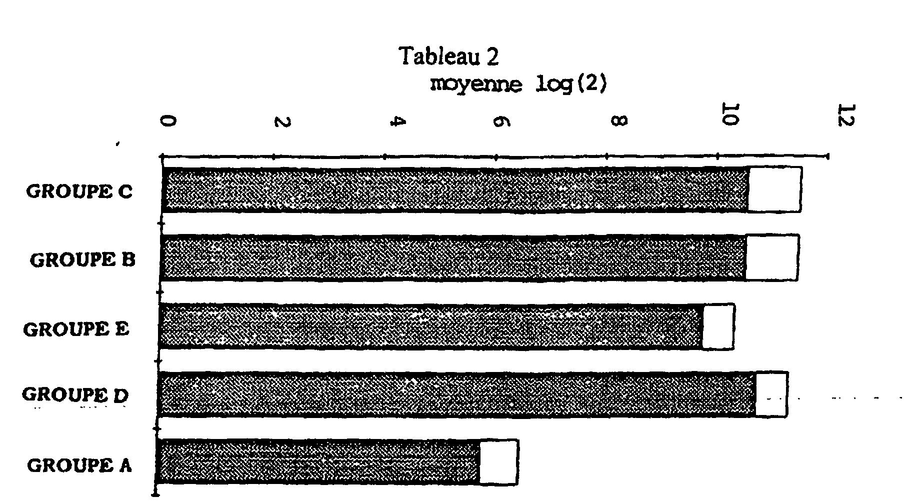

Les titres d'anticorps neutralisants dans les sérums des souris sont présentés en log2 de la dilution la plus forte induisant l'inhibition de l'hémagglutination.

Ces résultats montrent clairement le rôle d'adjuvant joué par le DC chol. En effet, le taux d'anticorps anti-HA est nettement plus élevé pour les lots de souris ayant reçu du DC chol comparativement aux souris ayant reçu le vaccin sans adjuvant (Groupe A).

Il est important de noter que les taux d'anticorps neutralisants dans les groupes B, C, D et E sont supérieurs au taux d'anticorps neutralisants dans le groupe A même après une injection unique des différentes compositions vaccinales. Ces titres augmentent encore légèrement et se stabilisent après la deuxième injection. Les résultats obtenus après la 3ème injection sont sensiblement égaux à ceux de la 2ème injection (non représentés).

Des essais réalisés avec un vaccin grippe trivalent comprenant la souche A Texas, la souche B Panama et la souche A Beijing ont également démontré le pouvoir adjuvant du DC chol.

### Exemple 10 : Mise en évidence de l'induction de cellules T cytotoxiques

On prélève à J51 les cellules spléniques des souris de chacun des groupes mentionnés à l'exemple 8.

Ces cellules effectrices sont restimulées in vitro en présence de cellules stimulantes syngéniques infectées par le virus de la souche A/ Singapour correspondant au vaccin testé. La fonction cytotoxique spécifique de ces cellules stimulées est mise en évidence en utilisant comme cellules cibles la lignée de mastocytomes P815 sensibilisés par incubation avec un peptide épitope CTL de l'hémagglutinine du virus (réponse spécifique contre la HA) ou avec un peptide épitope CTL de la nucléoprotéine du virus (réponse spécifique contre la NP). La lyse non spécifique (bruit de fond) est mesurée sur des cellules P815 non sensibilisées ou sensibilisées avec un peptide épitope CTL du virus HIV (V3MN).

La lyse des cellules cibles est mesurée par une technique radioactive basée sur le chargement des cellules cibles en Cr-51 et sur le relargage de ce radioélément lors de la lyse cellulaire.

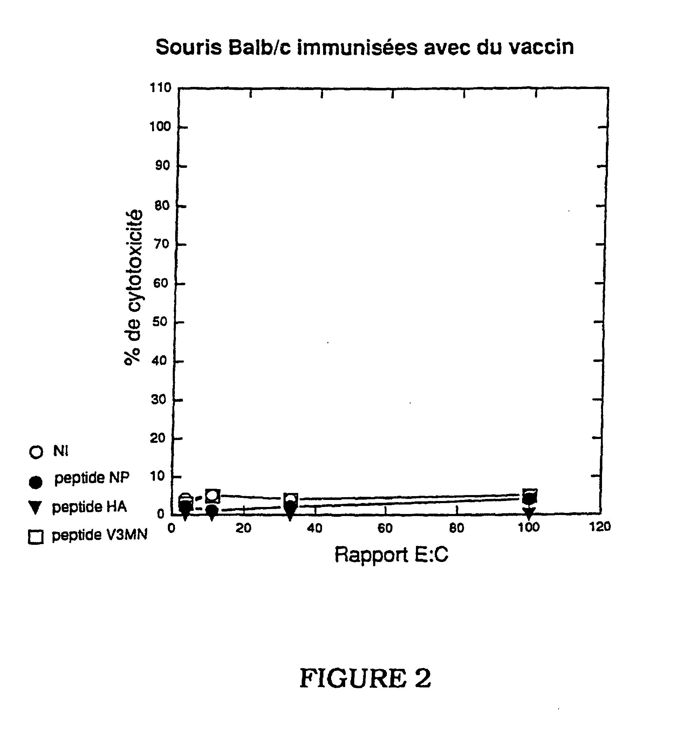

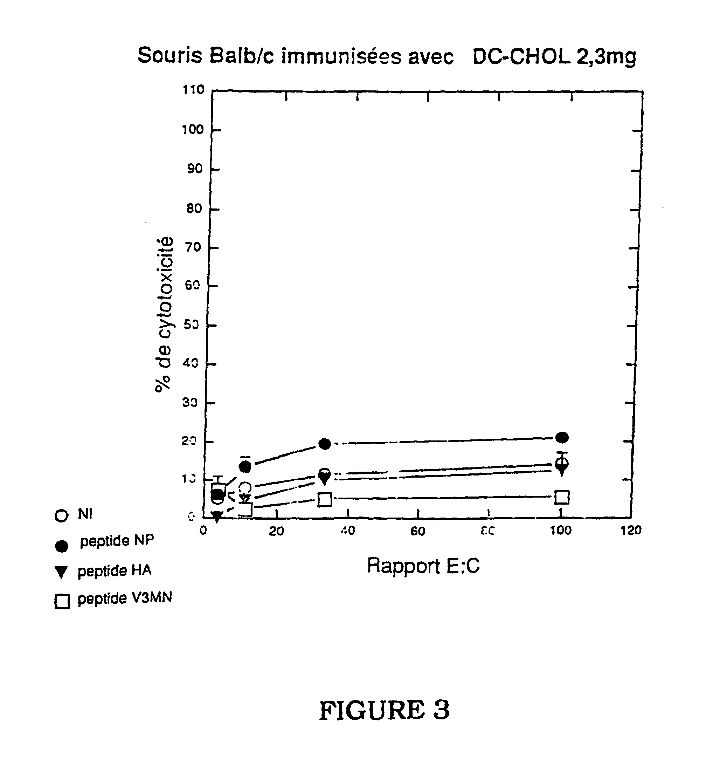

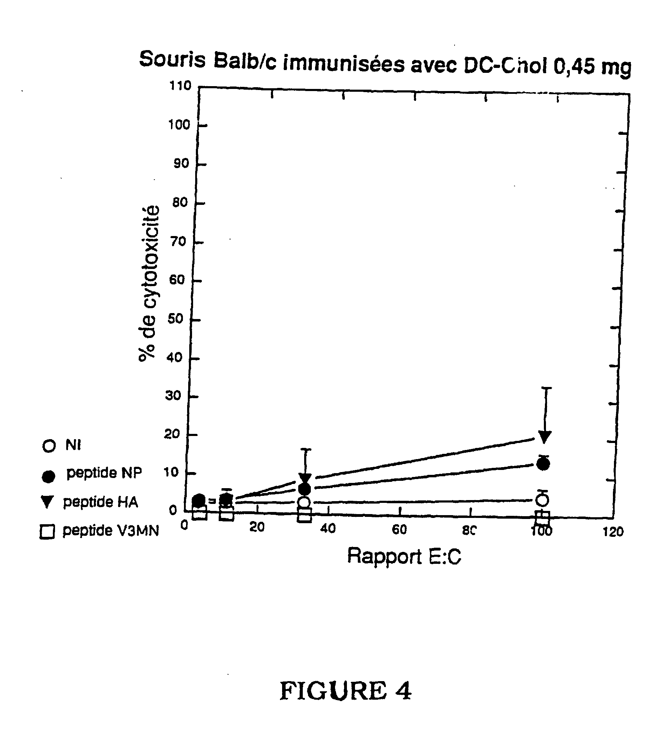

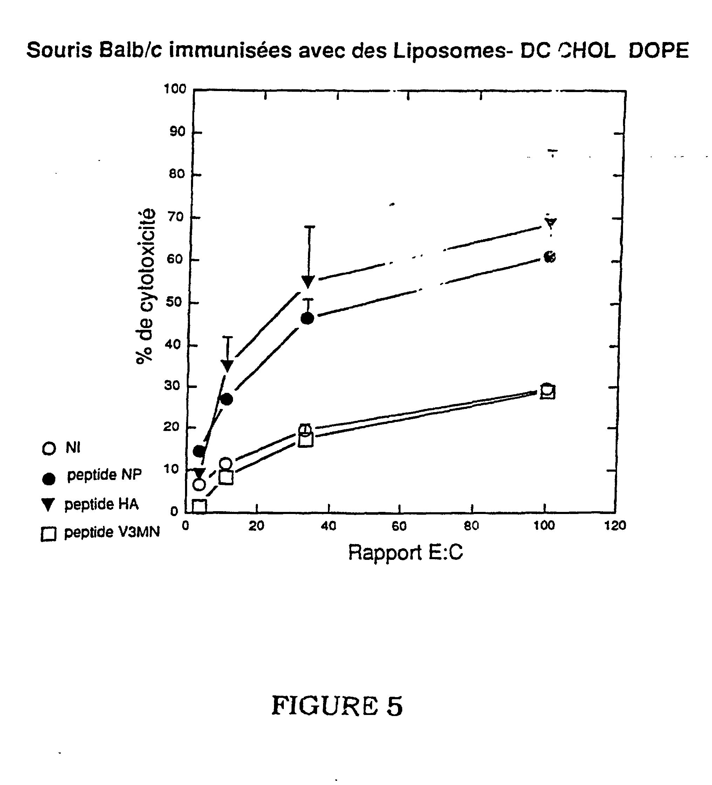

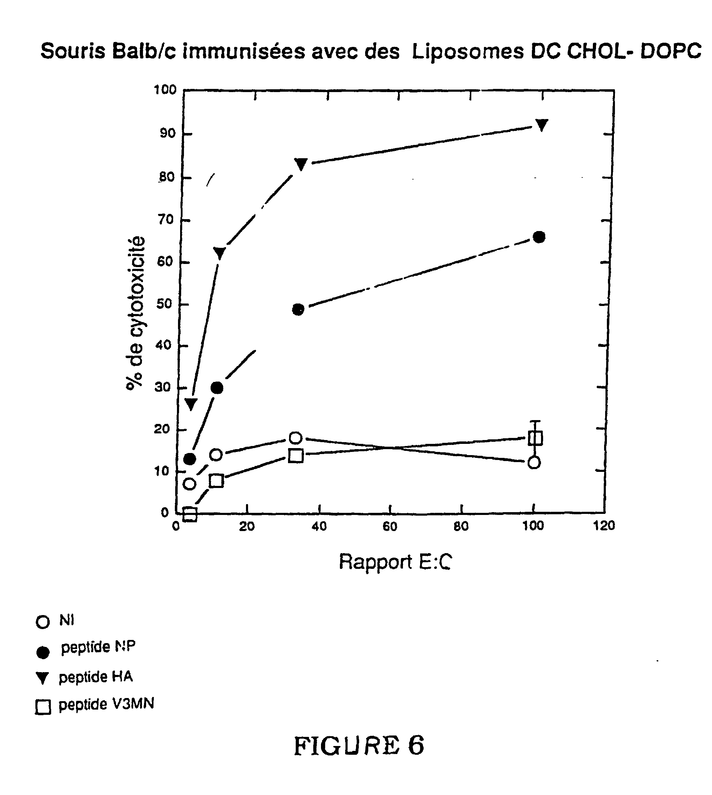

Les résultats présentés sur les figures 2 à 6 qui illustrent le pourcentage de cytotoxicité en fonction du rapport cellules effectrices sur cellules cibles pour chacun des groupes de souris testés, montrent qu'il est particulièrement avantageux d'utiliser une composition liposomale de DC chol en particulier en association avec un lipide neutre, car celle-ci permet d'induire des lymphocytes T cytotoxiques spécifiques en plus de la réponse immunitaire de type humoral obtenue grâce à l'action adjuvante du DC chol.

### Exemple 11 : Etude de la réponse immunitaire en fonction de la dose de DCchol utilisée

On réalise des compositions vaccinales de 300 µl comportant chacune du vaccin grippe monovalent de la souche A/Singapour et contenant soit 15 soit 5 µg de HA. associé à des liposomes de DC chol/DOPC à une concentration variable. La préparation de la suspension liposomale est réalisée de façon similaire à celle de l'exemple 4, en remplaçant les 18 mg de dioleoylphosphatidyléthanolamine (DOPE) par 13, 5 mg de dioleoyl phosphatidylcholine (DOPC) et en reprenant le film lipidique obtenu par une quantité d'eau variable selon la concentration en DC chol souhaitée.

On injecte les compositions vaccinales obtenues à des groupes de 5 souris Balb/c femelles de 8 à 10 semaines à J 0 et à J 28. On prélève les sérums à J 42 et on dose les Anticorps anti-HA par la technique d'inhibition de l'agglutination (IHA).

On teste ainsi les compositions suivantes :
- 15 µg de HA + 0 µg de DC chol
   15 µg de HA + 400 µg de DC chol
- 5 µg de HA + 0 µg de DC chol
   5 µg de HA + 25 µg de DC chol
   5 µg de HA + 50 µg de DC chol
   5 µg de HA + 100 µg de DC chol
   5 µg de HA + 200 µg de DC chol
   5 µg de HA + 300 µg de DC chol
   5 µg de HA + 400 µg de DC chol

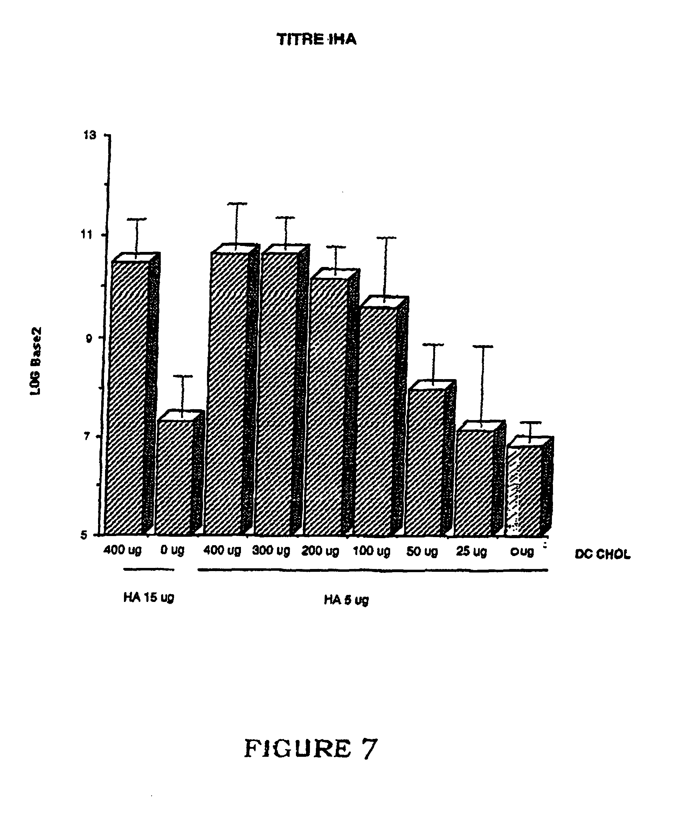

Les résultats exprimés en log base 2 de la dilution la plus forte induisant l'inhibition de l'hémagglutination sont représentés sur la figure 7 et montrent que ce n'est qu'au-dessus de 100 µg de DC chol/dose que l'on bénéficie du maximum de l'effet adjuvant du DC chol.

### Exemple 12 : Mise en évidence de l'induction de différents isotypes d'anticorps

On réalise une étude comparative des anticorps induits chez 3 groupes de 5 souris adultes BALB/c, femelles de 8 à 10 semaines, ayant reçu 2 injections sous-cutanées effectuées à J 0 et J 28 avec les compositions vaccinales suivantes :
- 1er groupe :: 0,3 ml de vaccin grippe monovalent souche A/Singapour dilué à 5 µg de HA dans 0,3 ml de PBS,
- 2ème groupe :: 0,3 ml de vaccin grippe monovalent souche A/Singapour dilué à 5 µg de HA adjuvé avec 0,1 mg d'hydroxyde d'aluminium,
- 3ème groupe :: 0,3 ml de composition vaccinale obtenue selon l'exemple 11 et contenant 5 µg de HA et 400 µg de DC chol associé à du DOPC.

On prélève les sérums des souris à J 28 et à J 42 et on dose par la technique ELISA les IgG1 et IgG2 produites.

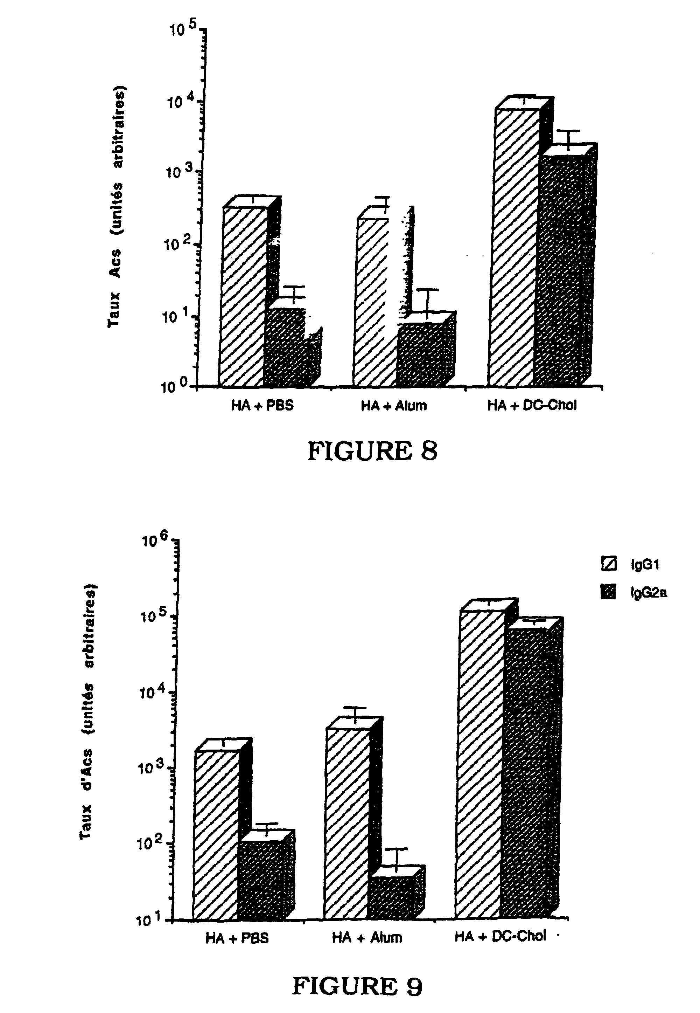

Les résultats obtenus lors de la réponse primaire sont représentés sur la figure 8 et ceux obtenus lors de la réponse secondaire sont représentés sur la figure 9.

Ces résultats illustrent l'effet adjuvant du DC chol tant lors de la réponse primaire que lors de la réponse secondaire, comparativement à l'hydroxyde d'aluminium qui est un adjuvant de l'art antérieur. On remarque, en outre, la forte augmentation des IgG2a produites, ce qui signifie que le DC chol agit en favorisant le développement des lymphocytes de type TH1.

### Exemple 13 : Mise en évidence de l'action du DC chol chez des souris âgées

On réalise exactement la même expérience qu'à l'exemple 12, mais cette fois, les souris testées sont des souris de 20 - 22 mois qu'il est plus difficile de stimuler.

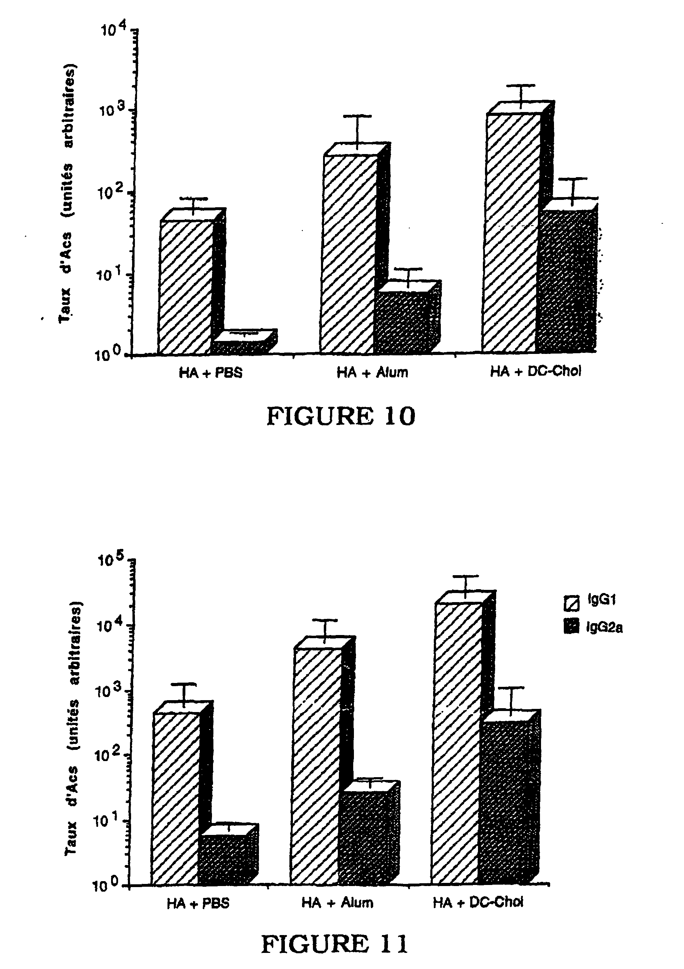

Les résultats obtenus lors de la réponse primaire sont représentés sur la figure 10 et ceux de la réponse secondaire sur la figure 11.

Ici encore, le DC chol a un effet d'adjuvant tant pour la réponse primaire que pour la réponse secondaire et notamment vis-à-vis de l'induction d'anticorps IgG2a.

### Exemple 14 : Taux IHA des souris adultes et des souris âgées

Lors des tests réalisés aux exemples 12 et 13, on détermine chez les souris immunisées les anticorps neutralisants par le test d'inhibition de l'hémagglutination (IHA).

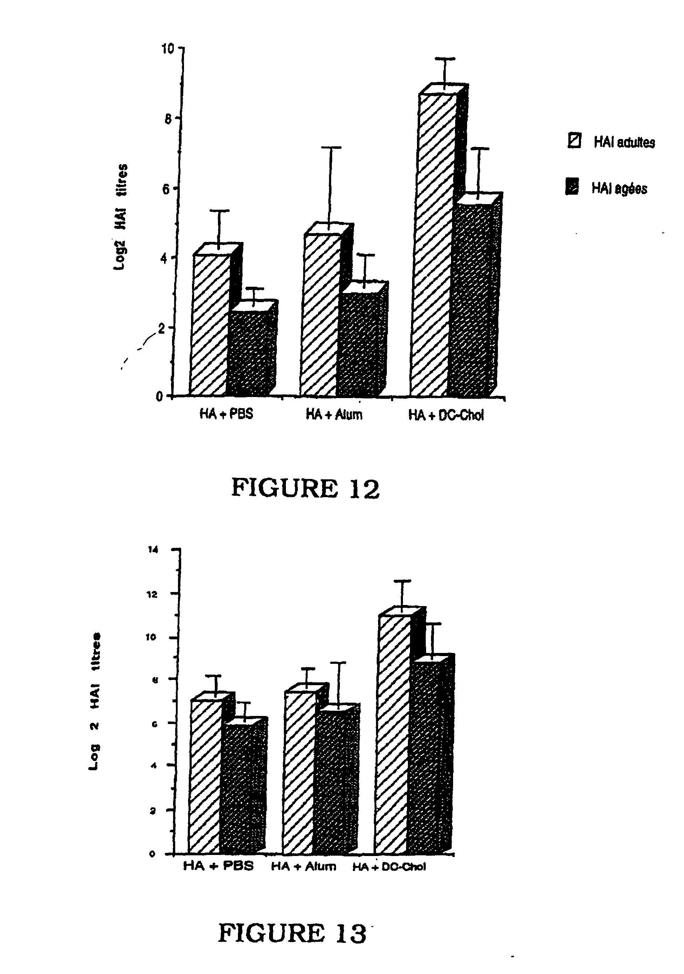

Les résultats obtenus lors de la réponse primaire sont illustrés sur la figure 12 et ceux de la réponse secondaire sur la figure 13. Les titres obtenus, exprimés en log base 2 de la dilution la plus forte inhibant l'hémagglutination, montrent bien le rôle adjuvant des liposomes DC chol/DOPC chez les souris adultes comme chez les souris âgées.

### Exemple 15 : Immunisation par voie intranasale

On prépare, comme cela a été décrit à l'exemple 11, des compositions vaccinales de 50 µl cette fois, comprenant 5 µg de HA (sous forme de vaccin grippe monovalent, souche A/Singapour) combinés avec 200 µg de DC chol présents sous forme de liposomes DC chol/DOPC (dans une proportion massique de 1 pour 4).

On immunise par voie intranasale 2 groupes de 4 souris BALB/c deux fois à 4 semaines d'intervalle.

Le 1er groupe de souris (G₁) reçoit 50 µl de la composition vaccinale contenant 200 µg de DC chol alors que le second groupe (G₂) reçoit 50 µl du même vaccin grippe monovalent mais sans adjuvant. On analyse les réponses immunes de chaque groupe par dosage ELISA des sérums prélevés 3 semaines après l'immunisation de rappel.

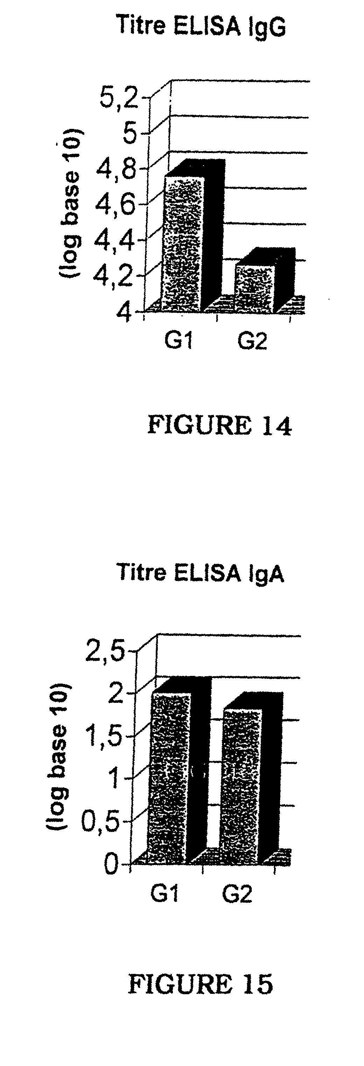

Les résultats, exprimés en log base 10 du titre ELISA, sont représentés sur la figure 14 pour ce qui concerne les IgG sériques et sur la figure 15 pour ce qui concerne les IgA sériques.

On remarque ainsi que, dans un protocole d'administration muqueux strict, les liposomes DC chol/DOPC permettent d'augmenter les réponses immunes locales et générales d'au moins un facteur 2.

Le rôle adjuvant joué par les liposomes DC chol/DOPC dans les réponses immunes générales a également été observé lorsqu'au lieu de mesurer les titres IgG et IgA par ELISA, on a mesuré les titres en anticorps anti-HA par IHA ; l'augmentation de la réponse immune locale a également été observée lors d'un comptage par ELISPOT du nombre de cellules sécrétrices d'IgG et du nombre de cellules sécrétrices d'IgA dans les poumons des souris immunisées, ainsi que lors de la détermination du rapport entre le taux d'IgG (respectivement d'IgA) spécifiques et le taux d'IgG (respectivement d'IgA) totales mesurées par ELISA dans les lavages nasopharyngés des souris immunisées.

### Exemple 16 : Immunisation par administration combinée en sous-cutané et par voie intranasale

On immunise 2 groupes de 4 souris BALB/c deux fois à 4 semaines d'intervalle avec, à chaque fois, les compositions vaccinales suivantes :
- Groupe 3 (G₃) :
   - une composition vaccinale de 300 µl injectée en sous-cutané comprenant 4 µg de HA (sous forme de vaccin grippe monovalent souche A/Singapour) combinés avec 6 µg de DC chol (sous forme de liposomes DC chol/DOPC) et,
   - une composition vaccinale de 50 µl administrée par voie intranasale comprenant 0,25 µg de HA (sous forme de vaccin grippe monovalent souche A/Singapour) combinés avec 6 µg de DC chol (sous forme de liposomes DC chol/DOPC),
- Groupe 4 (G₄):
   - une composition vaccinale de 300 µl injectée en sous-cutané comprenant 4 µg de HA combinés avec 200 µg de DC chol et,
   - une composition vaccinale de 50 µl administrée par voie intranasale comprenant 0,25 µg de HA combinés avec 200 µg de DC chol.

On prélève les sérums des souris immunisées 3 semaines après l'administration de rappel et on dose, pour chacune, le taux d'anticorps neutralisants par la technique IHA d'inhibition de l'hémagglutination.

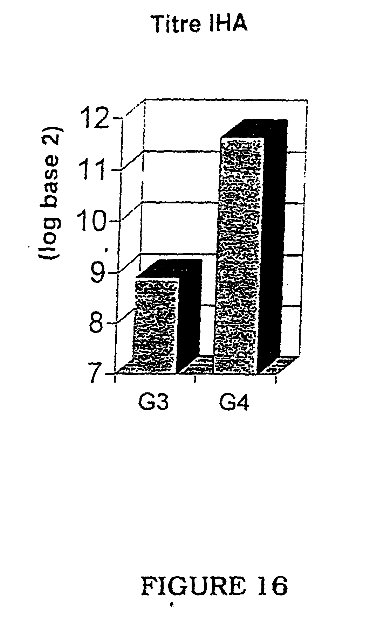

Les résultats obtenus, exprimés en log base 2 du titre, sont représentés sur la figure 16 et montrent que, dans un protocole d'administration mixte combinant les voies sous-cutanée et intranasale, le DC chol a un effet adjuvant et que l'augmentation de la dose de DC chol de 6 à 200 µg permet d'accroître la réponse immune.

Un effet dose identique a pu être observé lorsque, au lieu. de mesurer le taux d'anticorps anti-HA par la technique IHA, on a mesuré les IgG sériques par la technique ELISA ou la quantité de cellules sécrétrices d'IgG et la quantité de cellules sécrétrices d'IgA dans les poumons des souris immunisées (par la technique ELISPOT), ou le rapport entre les quantités d'IgG (respectivement IgA) spécifiques et les quantités d'IgG (respectivement IgA) totales dans les lavages naso-pharyngés des souris immunisées.

Des résultats similaires à ceux décrits aux exemples 11 à 16 ont été obtenus en remplaçant la DOPC par de la DOPE, ainsi qu'en faisant varier le rapport DC chol/lipide neutre, en maintenant la proportion molaire de lipide neutre associé au DC chol d'au moins 20 %.

## Revendications

1. Utilisation d'un composé amphipathique comprenant un groupement lipophile dérivé d'un stérol lié à un groupement cationique pour la fabrication d'une composition vaccinale.

2. Utilisation selon la revendication 1, **caractérisée en ce que** le groupement lipophile est un dérivé de cholestérol.

3. Utilisation selon une des revendications précédentes, **caractérisée en ce que** le groupement cationique est un ammonium quaternaire ou une amine protonable.

4. Utilisation selon une des revendications précédentes **caractérisée en ce que** le groupement lipophile est relié au groupement cationique par une liaison ester, éther, amide ou carbamoyle.

5. Utilisation selon une des revendications précédentes, **caractérisée en ce que** le groupement lipophile est séparé du groupement cationique par une chaîne alkylique ramifiée ou non comprenant de 1 à 20 atomes de Carbone.

6. Utilisation selon une des revendications précédentes, **caractérisée en ce que** le composé amphipathique est choisi parmi les composés suivants :
- l'iodure de cholestéryl-3β-carboxamidoéthylènetriméthylammonium,
- la cholestéryl-3β-carboxyamidoéthylènamine,
- l'iodure de cholestéryl-3β-oxysuccinamidoéthylènetriméthylammonium,
- le 3β-[N-(N',N'-diméthylaminoéthane)-carbamoyl]-cholestérol,
- le 3β-[N-(polyéthylènamine)-carbamoyl]-cholestérol.

7. Utilisation de 3β-[N-(N',N'-diméthylaminoéthane)-carbamoyl]-cholestérol pour la fabrication d'une composition vaccinale.

8. Utilisation selon une des revendications précédentes, **caractérisée en ce que** le composé amphipathique est associé à un lipide neutre.

9. Utilisation selon la revendication 8 **caractérisée, en ce que** la proportion de lipide neutre associé est d'au moins 20 %.

10. Utilisation selon une des revendications 8 et 9, **caractérisée en ce que** le lipide neutre est la dioleoyl phosphatidyléthanolamine (DOPE) ou la dioleoyl phosphatidylcholine (DOPC).

11. Utilisation selon une des revendications précédentes, **caractérisée en ce que** le composé amphipathique est dispersé dans un environnement aqueux sous forme de liposomes.

12. Utilisation d'un composé amphipathique comprenant un groupement lipophile dérivé d'un stérol lié à un groupement cationique pour la fabrication d'un adjuvant destiné à une administration vaccinale.

13. Utilisation selon la revendication 12, **caractérisée en ce que** ledit composé amphipathique est le 3β-[N-(N',N'-diméthylaminoéthane)-carbamoyl]cholestérol.

14. Utilisation selon une des revendications 12 ou 13, **caractérisée en ce que** ledit composé amphipathique est associé à un lipide neutre.

15. Utilisation selon une des revendications précédentes, **caractérisée en ce que** ladite composition vaccinale est destinée à être administrée à des mammifères.

16. Utilisation selon une des revendications précédentes, **caractérisée en ce que** ladite composition vaccinale est destinée à être administrée par voie infra-nasale.

17. Composition vaccinale comprenant au moins un antigène, **caractérisée en ce qu'**elle comprend en outre au moins un composé amphipathique possédant un groupement lipophile dérivé d'un stérol lié à un groupement cationique.

18. Composition vaccinale selon la revendication 17, **caractérisée en ce que** ledit groupement lipophile est un dérivé de cholestérol.

19. Composition vaccinale selon une des revendications 17 ou 18, **caractérisée en ce que** ledit composé amphiphatique est le 3β-[N-(N',N'-diméthylaminoéthane)-carbamoyl]cholestérol.

20. Composition vaccinale selon une des revendications 17 à19, **caractérisée en ce que** ledit composé amphiphathique se présente sous forme de liposomes incluant au moins un antigène.

21. Composition vaccinale selon une des revendications 17 à 20, **caractérisée en ce que** ledit composé amphipathique est associé à un lipide neutre.

22. Composition vaccinale selon une des revendications 17 à 21, **caractérisée en ce qu'**elle comprend au moins un antigène du virus de la grippe.

## Claims

1. Use of an amphipathic compound comprising a lipophilic group derived from a sterol linked to a cationic group, for the production of a vaccine composition.

2. Use according to Claim 1, **characterized in that** the lipophilic group is a cholesterol derivative.

3. Use according to either of the preceding claims, **characterized in that** the cationic group is a quaternary ammonium or an amine which can be protonated.

4. Use according to one of the preceding claims, **characterized in that** the lipophilic group is attached to the cationic group via an ester, ether, amide or carbamoyl link.

5. Use according to one of the preceding claims, **characterized in that** the lipophilic group is separated from the cationic group by a branched or unbranched alkyl chain comprising from 1 to 20 carbon atoms.

6. Use according to one of the preceding claims, **characterized in that** the amphipathic compound is selected from the following compounds:
- cholesteryl-3β-carboxamidoethylenetrimethylammonium iodide,
- cholesteryl-3β-carboxamidoethylenamine,
- cholesteryl-3β-oxysuccinamidoethylenetrimethylammonium iodide,
- 3β-[N-(N',N'-dimethylaminoethane)carbamoyl]-cholesterol,
- 3β-[N-(polyethylenamine)carbamoyl]cholesterol.

7. Use of 3β-[N-(N',N'-dimethylaminoethane)-carbamoyl]cholesterol for the production of a vaccine composition.

8. Use according to one of the preceding claims, **characterized in that** the amphipathic compound is combined with a neutral lipid.

9. Use according to Claim 8, **characterized in that** the proportion of neutral lipid combined is at least 20%.

10. Use according to either of Claims 8 and 9, **characterized in that** the neutral lipid is dioleoylphosphatidylethanolamine (DOPE) or dioleoylphosphatidylcholine (DOPC).

11. Use according to one of the preceding claims, **characterized in that** the amphipathic compound is dispersed in an aqueous environment in the form of liposomes.

12. Use of an amphipathic compound comprising a lipophilic group derived from a sterol linked to a cationic group, for the production of an adjuvant intended for a vaccine administration.

13. Use according to Claim 12, **characterized in that** the said amphipathic compound is 3β-[N-(N',N'-dimethylaminoethane)carbamoyl]cholesterol.

14. Use according to either of Claims 12 and 13, **characterized in that** the said amphipathic compound is combined with a neutral lipid.

15. Use according to one of the preceding claims, **characterized in that** the said vaccine composition is intended to be administered to mammals.

16. Use according to one of the preceding claims, **characterized in that** the said vaccine composition is intended to be administered by the intranasal route.

17. Vaccine composition comprising at least one antigen, **characterized in that** it comprises, in addition, at least one amphipathic compound possessing a lipophilic group derived from a sterol linked to a cationic group.

18. Vaccine composition according to Claim 17,
**characterized in that** the said lipophilic group is a cholesterol derivative.

19. Vaccine composition according to either of Claims 17 and 18, **characterized in that** the said amphipathic compound is 3β-[N-(N',N'-dimethylaminoethane)carbamoyl]cholesterol.

20. Vaccine composition according to one of Claims 17 to 19, **characterized in that** the said amphipathic compound takes the form of liposomes including at least one antigen.

21. Vaccine composition according to one of Claims 17 to 20, **characterized in that** the said amphipathic compound is combined with a neutral lipid.

22. Vaccine composition according to one of Claims 17 to 21, **characterized in that** it comprises at least one influenza virus antigen.

## Patentansprüche

1. Verwendung einer amphipatischen Verbindung mit einer von einem Sterin abgeleiteten lipophilen Gruppe, die an eine kationische Gruppe gebunden ist, zur Herstellung einer Impfstoffzusammensetzung.

2. Verwendung nach Anspruch 1, **dadurch gekennzeichnet, daß** es sich bei der lipophilen Gruppe um ein Cholesterinderivat handelt.

3. Verwendung nach einem der vorhergehenden Ansprüche, **dadurch gekennzeichnet, daß** es sich bei der kationischen Gruppe um ein quaternäres Ammonium oder ein protonierbares Amin handelt.

4. Verwendung nach einem der vorhergehenden Ansprüche, **dadurch gekennzeichnet, daß** die lipophile Gruppe über eine Ester-, Ether-, Amidoder Carbamoylbindung an die kationische Gruppe gebunden ist.

5. Verwendung nach einem der vorhergehenden Ansprüche, **dadurch gekennzeichnet, daß** die lipophile Gruppe von der kationischen Gruppe durch eine gegebenenfalls verzweigte Alkylkette mit 1 bis 20 Kohlenstoffatomen getrennt ist.

6. Verwendung nach einem der vorhergehenden Ansprüche, **dadurch gekennzeichnet, daß** die amphipatische Gruppe unter den folgenden Verbindungen ausgewählt ist:
- Cholesteryl-3β-carboxamidoethylentrimethylammoniumiodid,
- Cholesteryl-3β-carboxamidoethylenamin,
- Cholesteryl-3β-oxysuccinamidoethylentrimethylammoniumiodid,
- 3β-[N-(N',N'-Dimethylaminoethan)carbamoyl]-cholesterin,
- 3β-[N-(Polyethylenamin)carbamoyl]cholesterin.

7. Verwendung von 3β-[N-(N',N'-Dimethylaminoethan)-carbamoyl]cholesterin zur Herstellung einer Impfstoffzusammensetzung.

8. Verwendung nach einem der vorhergehenden Ansprüche, **dadurch gekennzeichnet, daß** die amphipatische Verbindung mit einem neutralen Lipid assoziiert ist.

9. Verwendung nach Anspruch 8, **dadurch gekennzeichnet, daß** der Anteil des assoziierten neutralen Lipids mindestens 20% beträgt.

10. Verwendung nach Anspruch 8 oder 9, **dadurch gekennzeichnet, daß** es sich bei dem neutralen Lipid um Dioleoylphosphatidylethanolamin (DOPE) oder Dioleoylphosphatidylcholin (DOPC) handelt.

11. Verwendung nach einem der vorhergehenden Ansprüche, **dadurch gekennzeichnet, daß** die amphipatische Verbindung in einem wäßrigen Milieu in Form von Liposomen dispergiert ist.

12. Verwendung einer amphipatischen Verbindung mit einer von einem Sterin abgeleiteten lipophilen Gruppe, die an eine kationische Gruppe gebunden ist, zur Herstellung eines Hilfsstoffs für eine Impfstoffverabreichung.

13. Verwendung nach Anspruch 12, **dadurch gekennzeichnet, daß** es sich bei der amphipatischen Verbindung um 3β-[N-(N',N'-Dimethylaminoethan)-carbamoyl]cholesterin handelt.

14. Verwendung nach Anspruch 12 oder 13, **dadurch gekennzeichnet, daß** die amphipatische Verbindung mit einem neutralen Lipid assoziiert ist.

15. Verwendung nach einem der vorhergehenden Ansprüche, **dadurch gekennzeichnet, daß** die Impfstoffzusammensetzung zur Verabreichung an Säugetiere vorgesehen ist.

16. Verwendung nach einem der vorhergehenden Ansprüche, **dadurch gekennzeichnet, daß** die Impfstoffzusammensetzung zur Verabreichung auf intranasalem Wege vorgesehen ist.

17. Impfstoffzusammensetzung mit mindestens einem Antigen, **dadurch gekennzeichnet, daß** sie außerdem mindestens eine amphipatische Verbindung mit einer von einem Sterin abgeleiteten lipophilen Gruppe, die an eine kationische Gruppe gebunden ist, enthält.

18. Impfstoffzusammensetzung nach Anspruch 17, **dadurch gekennzeichnet, daß** es sich bei der lipophilen Gruppe um ein Cholesterinderivat handelt.

19. Impfstoffzusammensetzung nach Anspruch 17 oder 18, **dadurch gekennzeichnet, daß** es sich bei der amphipatischen Verbindung um 3β-[N-(N',N'-Dimethylaminoethan)carbamoyl]cholesterin handelt.

20. Impfstoffzusammensetzung nach einem der Ansprüche 17 bis 19, **dadurch gekennzeichnet, daß** die amphipatische Verbindung in Form von Liposomen mit mindestens einem Antigen vorliegt.

21. Impfstoffzusammensetzung nach einem der Ansprüche 17 bis 20, **dadurch gekennzeichnet, daß** die amphipatische Verbindung mit einem neutralen Lipid assoziiert ist.

22. Impfstoffzusammensetzung nach einem der Ansprüche 17 bis 21, **dadurch gekennzeichnet, daß** sie mindestens ein Grippevirus-Antigen enthält.
